# EUROPEAN PATENT SPECIFICATION

(11) **EP 1 608 972 B1**
(45) Date of publication and mention of the grant of the patent: **14.11.2007**
(21) Application number: 03816517.1
(22) Date of filing: 31.03.2003
(51) Int. Cl.: G01N 33/543, C12N 11/08

(54) **METHOD FOR PREPARING PHOTOREACTIVE POLYMERS FOR IMMOBILIZING BIOMOLECULES THEREON**
VERFAHREN ZUR HERSTELLUNG VON PHOTOREAKTIVEN POLYMEREN ZUR IMMOBILISIERUNG VON BIOMOLEKÜLEN
PROCEDE POUR LA PREPARATION DE POLYMERES A REACTIFS A LA LUMIERE POUR L'IMMOBILISATION DE BIOMOLECULES

(43) Date of publication of application: 28.12.2005
(73) Proprietor: COUNCIL OF SCIENTIFIC AND INDUSTRIAL RESEARCH, New Delhi 110 001 (IN)
(72) Inventor: NAHAR, Pradip, Delhi 110 001 (IN); NAQVI, Azmi, Delhi 110 001 (IN)
(74) Representative: Smyth, Gyles Darren
(86) International application number: PCT/IN2003/000104
(87) International publication number: WO 2004/088316

(56) References cited:
- WO-A-02/14868
- DE-A- 19 818 360
- US-A- 3 959 078
- US-A- 5 858 653
- US-A- 5 986 066

## Description

### Field of the invention

This invention relates to a quick method for preparation of photoreactive polymers and immobilization of biomolecules onto these polymers. Photoreactive polymers are polymers, which become highly reactive when, exposed to light of specific wavelength and co-valently bind with the biomolecules. This method of immobilization is mild, simple and independent of pH and temperature. Advantage of the invention is that photo-immobilization technique can be easily manipulated and controlled.

### Background of the invention

Demand for immobilization of biomolecules onto organic or inorganic polymer has increased dramatically in recent years, particularly in the post genome era. Immobilized biomolecules have versatile applications in clinical laboratories, biosensors, membrane bioreactors, diagnostics, genomics, proteomics, drug screening, affinity chromatography and many other related fields. There are different approaches to immobilize a biomolecule onto an inert surface such as (i) to activate the polymer surface, (ii) to activate the biomolecule or (iii) activate both prior to immobilization. From the practical point of view, approach (i) is a better choice as in most of the cases the biomolecules are precious and sensitive towards chemicals and during activation some of them may be lost.

Biomolecules immobilized on the polymer surfaces are widely used in diagnostics, biosensors, membrane bioreactors, genomics, proteomics, drug screening, affinity chromatography and many other related fields [Krysteva, et. al., Covalent binding of enzymes to synthetic membrane containing acrylamide units, using formaldehyde, Biotechnol Appl. Biochem. 13: 106-111 (1991); Pandey, et.al., Amperometric enzyme sensor for glucose based on graphite paste-modified electrodes, Appl. Biochem. Biotechnol. 33:139-144 (1992)].

Demand for simple and rapid method of immobilization of biomolecules onto polymer surfaces continues to grow in the post genome era.

There are various methods available for immobilization of biomolecules onto the polymer surfaces. Covalent immobilization may results in better biomolecule activity, reduced nonspecific adsorption, and greater stability [Tiller, J. et. al., A novel efficient enzyme-immobilization reaction on NH2 polymers by means of L- ascorbic acid, Biotechnol. Appl. Biochem. 30, 155-162 (1992)]

There are number of ways for the covalent immobilization of biomolecules onto different polymer supports. Glucose oxidase, uricase, xanthine oxidase and penicillin acylase were immobilized on alkylamine glass or silica gel through glutaraldehyde coupling method [Guo, et. al., Immobilization of glucose oxidase and peroxidase and their application in flow injection analysis for glucose in serum, Appl. Biochem. Biotechnol. 23(1): 15-24 (1990); Nakamura, et. al., Immobilization of uricase on protamine bound to glass beads and its application to determination of uric acid, Anal. Biochem. 152(2): 386-390 (1986); Tawa, et. al., Immobilization of xanthine oxidase to controlled pore-glass. Application to high-performance liquid chromatography, Nucleic Acids Symp. Ser. 12: 107-110 (1983)]. Glucose oxidase, cholesterol oxidase and uricase immobilized on controlled pore glass were applied for determining glucose, cholesterol and urea respectively in serum.

Thionyl chloride-activated succinamidopropyl-controlled-pore glass was used to covalently immobilize rabbit anti- bovine IgG. [Stabel, et. al., Anti-IgG immobilized controlled pore glass. Thionyl chloride-activated succinamidopropyl glass as a covalent immobilization matrix, Appl. Biotechnol. 36(2):87-96(1992)]. Enzymes such as acetylcoenzyme

A synthetase was immobilized on CNBr-activated controlled pore glass. Activation of inert organic polymer surface such as polystyrene, polypropylene or polyethylene has been reported to occur through radiation graft polymerization or gaseous plasma technique [de Queiroz, et. al., Surface studies of albumin immobilized onto PE and PVC films, J. Biomater. Sci. Polym. 8: 667-681(1997); Siephia, et. al., Immobilization of enzymes on polypropylene bead surfaces by anhydrous ammonia gaseous plasma technique. J Biomed. Mater. Res. 22(5): 417-22 (1988)].

However, all these methods suffer from one or more shortcomings such as (i) they are tedious (ii) they are time consuming, (iii) they require hazardous chemicals, or (iv) require harsh reaction conditions.

The photolinker- mediated technique permits covalent binding of a biomolecule to solid surface under gentle reaction condition. This method is usually based on a photolinker having atleast two functional groups, one of which is essentially a photoactivable group.

There are different methods available for immobilization of biomolecule through a photolinker. Elsner, et. al. U.S. Pat. No. 5,427,779, describes a method for the activation of an inert surface by a two-ring heterocyclic photolinker such as psorlene by a photochemical reaction.

Jacobsen, et. al., U.S Pat. No. 6,033,784, discloses a method for photochemical activation of a polymer surface using quinone. Using primary amino containing quinone derivative, amino group is introduced onto polystyrene surface. However, these groups can not easily bind with the protein without addition of one or more activating reagent Preparation of such plates and photolinker are also time consuming and cumbersome.

Recently Nahar et. al. and Bora,et.al [Nahar, et. al., Light-induced activation of an inert surface for covalent immobilization of a protein ligand, Anal. Biochem. 294: 148-153 (2001); Nahar, P. US Patent Application No. 10/166,292, Method for photochemical activation of polymer surface and immobilization of biomolecules onto activated surface; Bora, et.al., Covalent immobilization of proteins onto photoactivated polystyrene microtiter plates (2002)] and later Naqvi et. al. [Naqvi, et. al., Introduction of functional groups onto polypropylene and polyethylene surfaces for immobilization of enzymes, Anal. Biochem. 306: 74-78 (2002)] published a simple, rapid and mild procedure for light- induced activation of inert polymers such as polystyrene, polypropylene and polyethylene using 1- fluoro 2-nitro -4- azido benzene. This procedure is used in the assay disclosed in WO-A-02/14868 (Scientific and Industrial Research Council).

However, in most of these photolinker- mediated procedures, surfaces were activated by a photochemical reaction whereas biomolecules were immobilized by a thermochemical reaction. Hence, biomolecule must have one or more functional group (s) for reaction with the activated surface resulting covalent linkage. In contrast, in photochemical immobilization technique, biomolecule can be immobilized irrespective of their functional group. There are very few reports on photochemical immobilization of biomolecule.

Sigrist, H. et al immobilized biomolecule on an inert surface by using a photolinker polymer made by reaction of BSA and 3-(triethylamine)-3-(m-isothiocyanophenyldiazirine). Photolinker polymer was then used to bind streptavidin as well as polystyrene surface simultaneously by exposing to light [Sigrist, et. al., Light dependent, Covalent immobilization of biomolecules on inert surface, Biotechnology 10: 1026-1028 (1992)]. However, preparation of this photolinker itself is a time consuming multistep procedure.

Guire, U.S Pat. No. 3,959,078 disclosed a method where immobilization of enzyme was carried out using a photolinker, 1- fluoro 2- nitro -4- azido benzene. In this method, amino- bearing matrix was activated by 1- fluoro 2- nitro -4- azido benzene in a thermochemical reaction at 37°C for 20 hours. In the subsequent step enzyme was immobilized in 16 hours onto the activated surface by a photochemical reaction. Disadvantage of this method is its long reaction time in both the steps. None of the above method gives the simple and rapid preparation of a photoreactive surface and rapid immobilization of biomolecule onto this photoreactive surface.

The following table gives the disadvantages of the prior art processes.

**Table I**

| **S.No** | **Prior art** | **Drawbacks** |
|---|---|---|
| 1 | C-H bearing matrix were activated photochemically with 1- fluoro-2- nitro-4-azido benzene followed by the attachment of the enzyme through the thermochemical reaction (Nahar et. al, 2001) | Method gave the limiting choice of the biomolecule i.e desired biomolecule must have amino group or other nucleophilic group for reaction with the activated surface. |
| 2 | Jorg Tiller (Tiller et. al, 1999) describes the method of activation of NH₂ polymer films by i) dipping the film in concentrated L-Ascorbic acid solution until the film had swollen visibly. Enzyme was then immobilized onto the activated film in 16 hours. ii) or the film was swollen in Dimethyl acetamide and subsequently placed in 10 ml of ice-cooled 0.5 M HCl, and 1 ml of 1M NaNO₂ solution was added dropwise. Enzyme was then immobilized at 4°C for 12 hours. | Activation procedure of Amino polymer film is cumbersome. Immobilization step is time consuming. |
| 3 | Biomolecule is immobilized on an inert surface by using a photolinker-polymer which was made by the reaction of BSA and 3-(triethylamine)-3-(m-isothiocyanophenyldiazirine). Photolinker-polymer was then used to bind streptavidin as well as polystyrene surface simultaneously when exposed to light. Sigrist, H. et al, (1992) | Preparation of this photolinker itself is a tedious and time consuming multistep procedure. |
| 4 | Guir, et. al., U. S Pat. # 5,002,582 describe a method for modification of alcohol bearing polymer in 16 hours at room temperature in dark using a photolinker. | Activation procedure is time consuming |
| 5 | U.S Pat. # 3,959,078 describes the modification of amino bearing polymer by a photolinker containing azido group in 20 hours. Immobilization on this polymer was carried out at 0°C- 15°C in 16 hours in a photochemical reaction using 40-watt tungsten lamp. | Both activation and immobilization procedure are time-consuming. |

### Objects of the invention

The main object of the invention is to provide a simple and quick method for the preparation of photoreactive polymers and rapid immobilization of biomolecules onto such polymers.

Yet another object of the invention is to provide photoreactive polymer by a microwave-mediated reaction of 1-fluoro-2- nitro-4- azidobenzene with the polymer. Another objective of the present invention is to provide a simple, mild and rapid method for immobilization of biomolecule irrespective of its functional group by a photochemical reaction.

Yet another object of the invention is to provide photoreactive polymer surface for efficient and rapid immobilization of biomolecule by UV light.

Yet another object of the invention is to make photoreactive polymer surface useful for light- induced immobilization of biomolecule required for chemical industry, pharmaceutical industry, diagnostics, separation technique and many other related fields.

Novelty of the present invention is in the rapid preparation of the photoreactive polymer, which takes one minute to ten minutes depending on the polymer.

### Summary of the invention

In order to achieve the objects of the present invention and overcoming the disadvantages of known prior art methods, a rapid, simple and efficient method is disclosed for preferably microwave- mediated preparation of photoreactive polymer and rapid light-induced immobilization of a biomolecule irrespective of its functional group onto the polymer.

Accordingly, the present invention provides A method for preparation of photoreactive polymers with molecules immobilized thereon, said method comprising:
(a) reacting a polymer with a nucleophilic group with a photolinker molecule dissolved in a suitable solvent,
(b) washing the polymer by a suitable solvent followed by drying the washed polymer at ambient temperature to obtain a photoreactive polymer,
(c) adding a molecule dissolved in a suitable buffer onto the photoreactive polymer,
(d) subjecting the mixture to a source of photoenergy for a period ranging from 2 minutes to 2 hours to immobilize the molecule on said photoreactive polymer, and
(e) washing the said polymer having immobilized molecule with a suitable buffer followed by drying the said polymer at the ambient temperature to obtain photoreactive polymers with molecules immobilized thereon.

In one embodiment of the invention, the photolinker has a thermochemical group selected from a group capable of forming a covalent bond with the nucleophilic group of the polymer.

In a further embodiment of the invention, the thermochemical group of the photolinker is selected from the group consisting of aldehyde, carbonyl, isothiocyanate, halide, and isocyanate.

In another embodiment of the invention, the photolinker has a photochemical group which is photoreactive and is capable of forming a covalent bond in a photochemical reaction with the molecule without addition of any other reagent.

In a further embodiment of the invention, the photochemical group of the photolinker is selected from the group consisting of precursors of carbene, precursors of nitrene and oxygen radical.

In yet another embodiment of the invention, the photolinker is 1- fluoro-2- nitro-4-azidobenzene (FNAB).

In another embodiment of the invention, the polymer comprises any polymer having amino, hydroxyl, thiol or any other reactive nucleophilic group such as, for example, silica gel, long chain alkyl amino controlled pore glass, polyvinyl alcohol, aminopolystyrene and alkylamino silica gel.

In another embodiment of the invention, the reaction in step (a) is carried out by exposing the mixture of photolinker, polymer and solvent to microwave radiation or by thermal incubation at 37°C, the microwave radiation being preferably carried out at a frequency of from 2000 Hz to 2600 Hz with a power output ranging from 100 watts to 1000 watts for a period ranging from 10 seconds to 20 minutes.

In another embodiment of the invention, the solvent used for washing the polymer obtained in step (a) comprises a solvent capable of dissolving the photolinker and its degraded products without distorting the polymer, such as methanol or ethanol.

In another embodiment of the invention, the solvent used in step (a) is selected from the group consisting of dimethyl formamide and toluene.

Preferably, the reaction in step (a) is carried out in the presence of a catalyst such as 30% KOH.

In yet another embodiment of the invention, the polymer is selected in the form of a bead, well, sheet, powder, stick, plate, strip or a tube.

In another embodiment of the invention, the molecule immobilized on the photoreactive polymer is selected from the group consisting of organic molecule, organic polymer, biomolecule and any molecule with C-H linkage.

In a further embodiment of the invention, the biomolecule is selected from the group consisting of protein, nucleic acids, carbohydrate, oligonucleotides, enzyme, antigen, antibody and peptide.

The enzyme is preferably horse radish peroxidase.

In yet another embodiment of the invention, the source of photoenergy is selected from the group consisting of UV light, sunlight, flush light and laser beam.

In a further embodiment of the invention, the UV light used as photoenergy source has a wavelength in the range of 300 nm to 400 nm and exposure to immobilize the molecule is carried out for a period in the range of 2 minutes to 2 hours.

The reaction between the nucleophilic group of the polymer and the photolinker is step (a) is preferably is carried out in a microwave oven in presence of a solvent.

The invention also relates to the use of a photoreactive polymer with a molecule immobilized thereon for diagnostics, affinity chromatography, proteomics, gemomics and drug screening.

### Brief description of the accompanying drawings

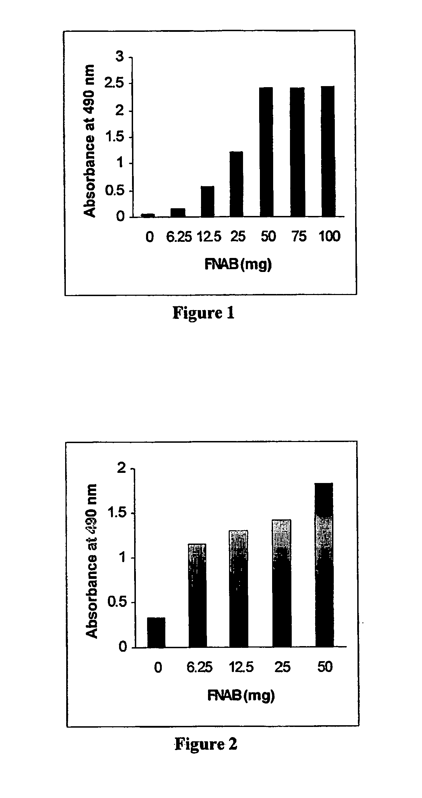

### Figure 1. Optimization of amount of FNAB for the preparation of photoreactive silica gel (Example 1)

Optimization of amount of FNAB for the preparation of photoreactive silica gel was done by adding varied amount of FNAB (0, 6.25, 12.5, 25, 50, 75 and 100 mg) in each reaction with 50 mg alkylamino silica gel and exposing them to microwave irradiation for 60 seconds.

Photoreactive alkylamino silica gel thus prepared in each reaction was checked by immobilizing HRP (1 µg) onto the support (50 mg) by a photochemical reaction. Absorbance was recorded after assaying the immobilized enzyme colorimetrically.

### Figure 2. Optimization of amount of FNAB for the preparation of photoreactive LCAA-CPG (Example 2)

Optimized amount of FNAB needed for the preparation of photoreactive LCAA-CPG was determined by changing concentrations of FNAB (0, 6.25, 12.5, 25 and 50 mg) in each reaction with 50 mg of LCAA-CPG and exposing them to microwave radiation for 60 seconds. Photoreactive LCAA-CPG thus prepared in each reaction was checked by photoirradiating HRP (1µg) with 50mg of support and assaying immobilized enzyme colorimetrically.

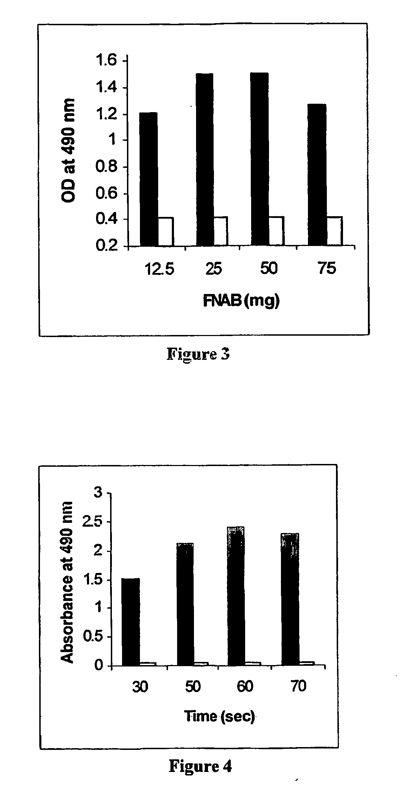

### Figure 3. Optimization of FNAB amount for the preparation of photoreactive PVA (Example 3)

Optimized amount of FNAB for the preparation of photoreactive PVA was determined by by adding varied amount of FNAB (12.5, 25, 50 and 75 mg) to the reaction mixture of 50 mg of PVA and 6.5 µl of 30% KOH and 5 ml toluene. The reaction mixtures were kept at room temperature for 1 hour. Photoreactive PVA thus prepared in each reaction was checked by irradiating HRP (16 µg) with 50 mg of the support. Absorbance was recorded after addition of substrate to the immobilized enzyme.

### Figure 4. Optimization of exposure time for microwave irradiation for making photoreactive alkylamino silica gel (Example 4)

Microwave exposure time for making photoreactive alkylamino silica gel was optimized by changing microwave exposure time (30, 50, 60 and 70 seconds) in the reaction of 50 mg alkylamino silica gel with 50 mg FNAB. Photoreactive surface thus prepared in each reaction was checked by irradiating 1 µg of HRP with 50 mg support and assaying the immobilized enzyme colorimetrically.

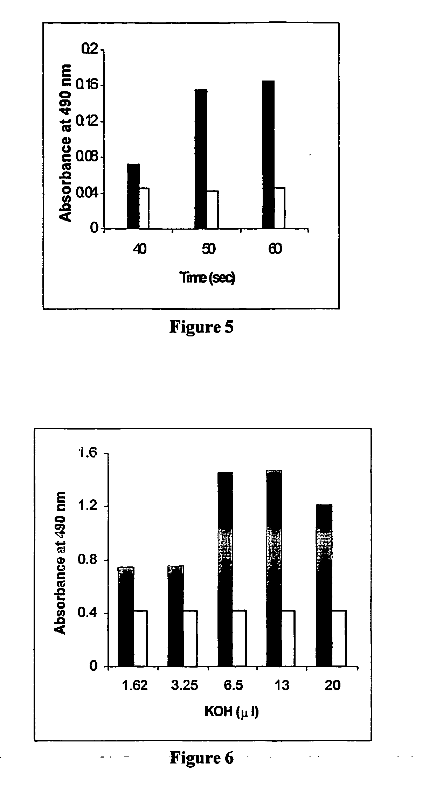

### Figure 5. Optimization of microwave exposure time for the activation of amino-polystyrene (Example 5)

Microwave exposure time for making photoreactive amino-polystyrene was optimized by changing microwave exposure time (40, 50 and 60 seconds) in each reaction of 20 mg amino-polystyrene with 20 mg FNAB. Photoreactive surface thus prepared in each reaction was checked by irradiating HRP (35 µg) with 20 mg of the support. Absorbance was recorded after addition of substrate to the immobilized enzyme.

### Figure 6. Optimization of KOH concentration for the preparation of photoreactive PVA (Example 6)

KOH concentration for the preparation of photoreactive PVA was optimized by adding different amount of 30 % KOH solution (1.62, 3.25, 6.5, 13 and 20 µl) to the reaction mixture of 50 mg PVA and 50 mg FNAB in 5 ml toluene. Photoreactive surface thus prepared in each reaction was checked by irradiating HRP (16 µg) with 50 mg of the support and assaying the immobilized enzyme colorimetrically.

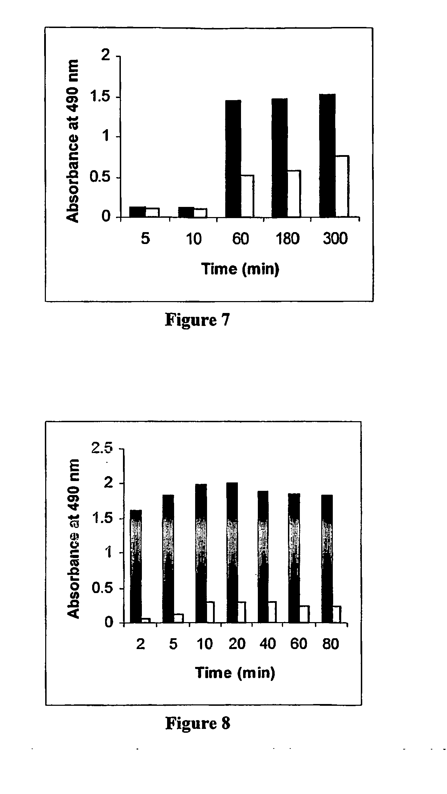

### Figure 7. Optimization of activation time for PVA at room temperature (Example 7)

Activation time for the preparation of photoreactive PVA was optimized by taking different incubation time (5,10, 60, 180 and 300 minutes) for each reaction mixture of 50 mg PVA, 50 mg FNAB in 5 ml toluene and 6.5 µl of 30% KOH. Photoreactive surface thus prepared in each reaction was checked by irradiating HRP (16 µg) with 50 mg of the support and assaying the immobilized enzyme colorimetrically.

### Figure 8. Optimization of time of irradiation for the immobilization of enzyme onto the alkylamino silica gel (Example 8)

Immobilization time was optimized by mixing HRP (1 µg) with 50 mg photoreactive alkylamino silica gel and changing the UV irradiation time (2, 5, 10, 20, 40, 60 and 80 minutes) for each reaction. Absorbance was recorded after addition of substrate to the immobilized enzyme.

### Figure 9. Optimisation of photoirradiation time for enzyme immobilization onto the photoreactive LCAA- CPG (Example 9)

Immobilization time was optimized by mixing 50 mg photoreactive LCAA- CPG with HRP (1 µg) and irradiating it by UV in different time (2, 10, 20, 40 and 60 minutes) for each reaction. Immobilized enzyme was then assayed colorimetrically after addition of substrate.

### Figure 10. Optimization of UV irradiation time for enzyme immobilization onto photoreactive PVA surface (Example 10)

Immobilization time was optimized by mixing 50 mg photoreactive PVA with HRP (16 µg) and irradiating it by UV in different time (5, 20, 30, 60,120 and 180 minutes) for each reaction. Absorbance was recorded after addition of substrate-dye buffer to the immobilized enzyme.

### Figure 11. Optimization of the enzyme concentration for its immobilization on photoreactive PVA (Example 11)

HRP concentration was optimized by taking different amount of enzyme (0.25 0.5, 1.0, 2.0, 4.0 8.0 and 16.0 µg) for each 50 mg photoreactive PVA and irradiating it for 30 minutes by UV light. Immobilized enzyme was then assayed colorimetrically after addition of substrate.

### Figure 12. Optimization of enzyme concentration for its immobilization onto microwave- activated photoreactive LCAA-CPG (Example 12)

HRP concentration was optimized by taking different amount of enzyme (0.2, 0.5, 1, 2 and 4 µg) for 50 mg photoreactive LCAA- CPG and irradiating it by UV light for 20 minutes. Absorbance was recorded after addition of substrate-dye buffer to the immobilized enzyme.

### Figure 13. Optimization of enzyme concentration for its immobilization onto microwave- activated alkylamino silica gel (Example 13)

Optimization of HRP concentration for 50 mg photoreactive alkylamino silica gel was carried out by taking different amount of enzyme (0.5, 1.0, 2.0, 4.0, and 6.0 µg) for each reaction and irradiating it for 20 minutes by UV light. Immobilized enzyme was then assayed colorimetrically after addition of substrate

### Detailed description of the invention

The present invention comprises a rapid and efficient method for modification of polymer surface for the introduction of latent reactive group onto it and immobilization of biomolecules onto said modified polymer surface by light energy. More specifically, polymer surface with latent photoreactive group is prepared rapidly by microwave-mediated reaction of 1- fluoro-2- nitro-4- azidobenzene with a polymer having a reactive nucleophilic group. The modified polymer (photoreactive polymer) thus prepared has photoreactive azido group, which under UV light generates highly reactive nitrene capable of binding with the biomolecule.

Concentration of photolinker plays an important role in preparing photoreactive polymer. Best result was obtained when the ratio of the photolinker (FNAB) and polymer is 1:1 (w/w). Further increase in FNAB did not increase the photoreactive group in a polymer. The efficacy of the photoreactive surface was determined by immobilizing HRP onto it and subsequently assaying the immobilized enzyme. When untreated surface (FNAB: 0 mg) was used, there was practically no immobilization of biomolecule onto it. (fig 1, fig 2 and fig 3).

Microwaves were found an excellenf tool for making photoreactive surface. Thus, photoreactive LCAA-CPG prepared in 70 microwave irradiation was found to give better results than the photoreactive surface obtained by prior art i.e 37°C for 20 hours (table 1).

Microwave radiation for 60 seconds was also found to give photoreactive aminosilica with good results (fig 4). For aminopolystyrene also 60 seconds microwave irradiation was sufficient for the reaction of the polymer and FNAB to produce photoreactive amino polystyrene. (Fig 5). In contrast to amino polymer, hydroxyl group bearing polymer needed a basic catalyst for the reaction with FNAB. Optimization of amount of KOH (catalyst) was carried out by performing the reaction of PVA and FNAB by stirring at room temperature for one hour in presence of toluene and different amount of KOH in aqueous solution. 6.5 µl of 30 % KOH was found as optimum for the reaction of PVA and FNAB (50 mg each) in presence of 5 ml toluene. (fig 6). The applicants also optimized the time required for making photoreactive PVA using KOH at room temperature. The optimum time was 60 minutes. (fig 7). However, comparable results were obtained by microwave irradiation in 10 minutes.

In photoactivation procedure PVA was activated by exposing FNAB coated PVA to UV light. The photoactivated PVA was then used to immobilize horse radish peroxidase at 37°C for 1 hour. In the invented procedure, photoreactive PVA was prepared by the reaction of PVA and FNAB by microwave irradiation (or by thermal incubation at 37°C). Horse radish peroxidase immobilization on this photoreactive PVA was then carried out by exposing them to UV light. Results in table 2 showed that photoreactive polymer (surface activated thermally) gave better immobilization of biomolecule than thermoreactive (surface activated by light) surface. Hence, photochemical immobilization procedure (thermochemical activation and photochemical immobilization) as described in this invented procedure is better option than thermochemical immobilization (photochemical activation and thermochemical immobilization) procedure.

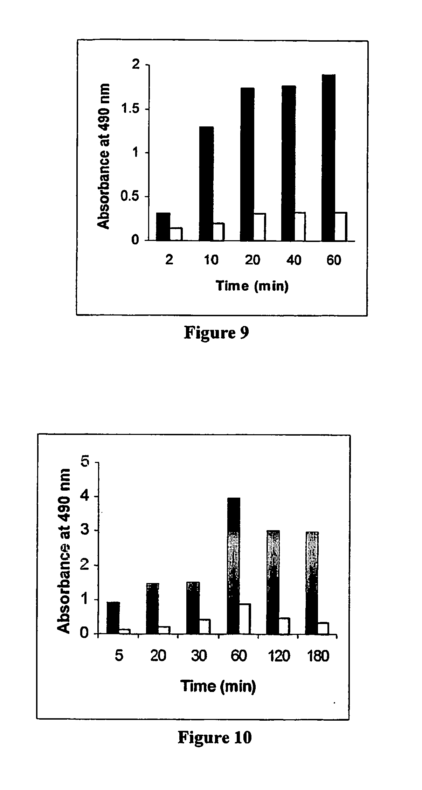

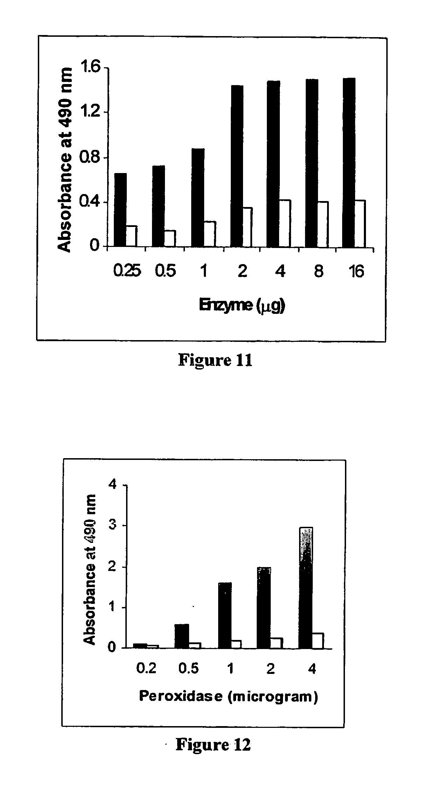

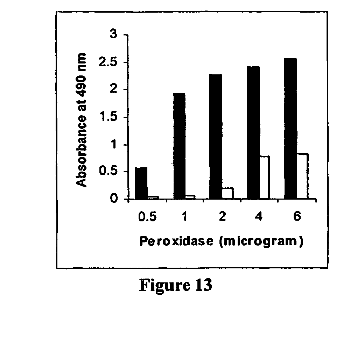

The applicants have optimized the concentration of linker (FNAB) and time of incubation with polymer (PVA) for the preparation of photoreactive polymer. In the second step, enzyme (HRP) was immobilized onto the polymer by its photoreactive group by light energy. Thus, immobilization of enzyme onto the alkylamino silica gel was carried out by UV light in a UV stratalinker in different time. The optimum time for photoimmobilization of enzyme on photoreactive alkylamino silica gel was found as 10 minutes. However, increase in UV irradiation time beyond 10 minutes did not increase immobilization (fig 8). Immobilization of HRP onto photoreactive LCAA-CPG was found optimum in 20 minutes (fig 9). However, photoreactive PVA requires 60 minutes of UV irrradiation for optimum immobilization of HRP. Further increase in UV irradiation time decreases the absorbance, which may be due to the deactivation of some immobilized enzymes. (fig 10). Enzyme concentration is also an important factor for immobilization. 2 µg HRP/ 50 mg photoreactive PVA was found optimum for the immobilization on PVA and further increase did not appreciable increase its immobilization (fig 11). However, optimum amount of HRP was 4 µg/ 50 mg support for immobilization on photoreactive LCAA-CPG (fig 12) and alkylamino silica gel (fig 13). Total active enzyme immobilized on 20 mg LCAA-CPG and 20 mg alkylamino silica gel was found 160 U and 120 U respectively.

Thus, in the present invention, this method of preparation of photoreactive supports is simple, rapid and overcomes drawbacks of the reported methods. Also, the immobilization of biomolecules onto the photoreactive surfaces are simple and fast.

The advantages of the process of the invention are given in the Table below:

**TABLE II**

| **PRESENT INVENTION AND ITS ADVANTAGES** | | |
|---|---|---|
| **S.No** | **Present Invention** | **Advantage** |
| 1 | The present invention provides a rapid, microwave-mediated method for preparation of photoreactive polymers capable of forming a covalent bond with the biomolecules irrespective of the functional group of the biomolecule. | The invented method can activate the amino- polymers in around 50 seconds and alcohol bearing polymers in around 10 minutes by microwave irradiation. Hence, overcomes the lengthy process of making photoreactive polymer. |
| 2 | The present invention also provides a rapid technique, which varies from 10 - 70 minutes for immobilization of biomolecules on to the photoreactive polymer. | In the present invention, the optimum time for immobilization of biomolecule is 10 -70 minutes, which is much less than the prior art (12-16 hours) using the same photolinker. 2) The activated surfaces have a potential application in chromatographic separations, genomics and proteomics where immobilized molecules are required. 3) 1-Fluoro 2-nitro- 4-azido benzene used as a photolinker can be made easily. |

Horse radish peroxidase, Long chain alkyl amine-Controlled pore glass (Normal diameter 500 A°, Mesh size: 80-120), o-phenylenediamine were purchased from Sigma, USA. Silica gel LR (100-200 mesh) was purchased from S.D fine chemicals. H₂O₂, methanol, di-methyformamide, toluene and 3-aminopropyltriethoxysilane, sodium chloride, di-sodium hydrogen orthophosphate, sodium dihydrogen ortho phosphate, citric acid were of analytical grade purchased from Merck, India. FNAB was prepared from 4-fluoro-3-nitroaniline by diazotization reaction as reported earlier [15]. Microwave mediated reactions were carried out in BPL Sanyo microwave oven operating at a frequency of 2450 Hz with a power output of 700 watts. Photo-immobilization was carried out at a wavelength of 365 nm in an U.V stratalinker; model 2400, (Stratagene, USA) fitted with five 15-watt tubes. All the solutions were freshly prepared in triple-distilled water before use. Phosphate Buffered Saline (PBS) was prepared by mixing 0.85 % NaCl to 0.01 M phosphate buffer (pH 7.2). Wash buffer was prepared by adding (0.1% Tween 20 in PBS). A freshly prepared substrate dye buffer contain 12 ml of citrate buffer (0.025 M citric acid and 0.5 M Na₂HPO₄.2H₂O, pH 5), 5 µl of H₂O₂ (30% w/v) and 4 mg of o-phenylenediamine.

### Example 1: Optimization of amount of 1-fluoro 2- nitro -4- azidobenzene for the preparation of photoreactive silica gel (figure 1)

5 g of dry (moisture free) silica gel mixed with 10 ml of 3-aminopropyltriethoxysilane and 80 ml toluene was stirred at 28°C for 3 hours. It was filtered and washed with methanol, water, methanol: water (1:1) and methanol respectively. The support was then dried and assayed for amino group by ninhydrin test. Positive test for ninhydrin confirm the linking of amino group to silica gel. Conical flask containing 50 mg alkylamino silica gel, 6.25 mg FNAB and 10 ml DMF was exposed to microwaves for 60 sec. After which the support from the conical flask was washed by methanol, dried and kept in petridish. FNAB concentration was optimized by doing the same experiments with different amounts of FNAB (12.5, 25, 50, 75 and 100 mg respectively). HRP (1µg / 80 µl of PBS) was then added to 50 mg of photoreactive silica gel and irradiated in an UV stratalinker at 365 nm for 20 minutes. The support was washed with washing buffer followed by the addition of 300 µl of substrate-dye buffer. The coloured solution was transferred to the respective polystyrene mictotiter wells and absorbance was recorded at 490 nm by an ELISA reader. A control experiment was carried out with untreated alkylamino silica gel (FNAB: 0 mg) in the similar way

### Example 2: Optimization of 1-fluoro 2- nitro -4-azidobenzene concentration for the preparation of photoreactive long chain alkylamine controlled pore glass (figure 2)

50 mg LCAA- CPG, 6.25 mg FNAB and 10 ml DMF were taken in a conical flask exposed to microwaves for 60 sec. FNAB concentration was optimized by doing the same experiment with different amounts of FNAB (12.5, 25 and 50 mg respectively).

Enzyme immobilization and its assay were carried out similarly as described in example 1. A control experiment was carried out with untreated LCAA- CPG (FNAB: 0 mg).

### Example 3: Optimization of 1-fluoro 2- nitro -4- azidobenzene concentration for preparation of photoreactive PVA (figure 3)

A conical flask containing 50 mg PVA, 50 mg FNAB, 6.5 µl of 30% KOH and 5 ml toluene were kept for stirring in dark at room temperature (28°C) for 1 hour. Three more conical flasks were taken and reaction mixtures were prepared similarly but with the FNAB concentration of 12.5, 25 and 75 mg respectively. After 1 hour, supports from conical flasks were washed by methanol, dried and kept in petridishes. HRP (16µg / 80 µl of PBS) was added to each photoreactive PVA (50 mg) and irradiated in UV stratalinker at 365 nm for 30 minutes. Enzyme was assayed similarly as described in example 1. A control experiment was carried out with untreated PVA beads in the similar way.

### Example 4: Optimization of microwave exposure time for the activation of alkylamino silica gel (figure 4)

Four conical flasks, each containing 50 mg alkylamino silica gel, 50 mg FNAB and 10 ml DMF were exposed to microwave for 30, 50, 60 and 70 seconds respectively. After the stipulated time, supports from each conical flask were washed separately by methanol, dried and kept on four petridishes. HRP was then immobilized similarly as described in example 1. Control experiment was carried out with untreated alkylamino silica gel beads in similar way.

### Example 5: Optimization of exposure time in microwave for the activation of amino-polystyrene (figure 5)

20 mg aminopolystyrene, 20 mg FNAB and 10 ml ethanol were taken in 50 ml conical flask and exposed to microwaves for 40 seconds. Another two experiments were carried out in the similar manner but with the microwave exposure time of 50 and 60 seconds respectively. After the stipulated microwave exposure time the support from the conical flasks were washed by ethanol, dried and kept on petridishes. HRP (35 µg / 80 µl of PBS) was added to each photoreactive aminopolystyrene (50 mg), and irradiated in UV stratalinker at 365 nm for 20 minutes. Enzyme was assayed similarly as described in example 1. A control experiment was carried out with untreated aminopolystyrene in the similar manner.

### Example 6: Optimization of KOH concentration for the thermoactivation of PVA (figure 6)

A conical flask containing 50 mg PVA, 50 mg FNAB, 1.62 µl of 30 % KOH and 5 ml toluene were kept in dark for 1 hour with continuous stirring at room temperature. Another four experiments were carried out similarly but with different quantity of 30 % KOH solution (3.25, 6.5, 13 and 20 µl respectively). After an hour the supports from the conical flasks were washed by methanol separately, dried and kept on petridishes. HRP (16 µg / 80 µl of PBS) was added to each photoreactive PVA (50 mg) and irradiated in UV stratalinker at 365 nm for 30 minutes. The supports were washed with washing buffer followed by the addition of 300 µl of substrate-dye buffer. The coloured solution was transferred to the respective polystyrene mictotiter wells and absorbance was recorded at 490 nm by an ELISA reader. A control experiment was carried out with untreated PVA beads in the similar way.

### Example 7: Optimization of time for thermal activation of PVA (figure 7)

Four conical flasks, each containing 50 mg PVA, 50 mg FNAB, 6.5 µl of 30% KOH and 5 ml toluene were kept in dark and stirred at room temperature for 5, 10, 60, 180 and 300 minutes respectively. HRP was immobilized on the photoreactive PVA and assayed as described in example 6. Control experiment was carried out with untreated PVA beads similarly.

### Example 8: Optimization of UV irradiation time for immobilization of enzyme onto the microwave activated photoreactive alkylamino silica gel (figure 8)

300 mg alkylamino silica gel was activated using 300 mg FNAB in 10 ml DMF by 70 seconds microwave irradiation. Six petri plates, each containing 50 mg photoreactive alkylamino silica gel and HRP (1µg / 80 µl of PBS) were irradiated in UV stratalinker at 365 nm for 2, 5, 10, 20, 40, 60 and 80 minutes respectively. The supports were washed with washing buffer followed by the addition of 300 µl of substrate-dye buffer. The coloured solution was transferred to the respective polystyrene microtiter wells and absorbance was recorded at 490 nm by an ELISA reader. A control experiment was carried out with untreated alkylamino silica gel in the similar way.

### Example 9: Optimization of time of irradiation for the immobilization of enzyme onto the microwave activated photoreactive LCAA- CPG (figure 9)

300 mg LCAA-CPG was activated using 300 mg FNAB in 10 ml DMF by 70 seconds microwave irradiation. Six petri plates, each containing 50 mg photoreactive LCAA- CPG and HRP (1µg / 80 µl of PBS) were irradiated in UV stratalinker at 365 nm for 2, 10, 20, 40 and 60 minutes respectively. Immobilized enzyme was assayed as described in example 8. A control experiment was carried out with untreated LCAA- CPG in the similar way.

### Example 10: Optimizing time of UV irradiation for enzyme immobilization onto the microwave activated photoreactive PVA (figure 10)

300 mg PVA was activated using 300 mg FNAB dissolved in 10 ml DMF by 10 minutes microwave irradiation. Six petri plates, each containing 50 mg photoreactive PVA mixed with HRP (16µg / 80 µl of PBS) was irradiated in UV stratalinker at 365 nm for 5, 20, 30, 60,120 and 180 minutes respectively. The supports were washed with washing buffer followed by the addition of 300 µl of substrate-dye buffer. The coloured solution was transferred to the respective polystyrene microtiter wells and absorbance was recorded at 490 nm by an ELISA reader. Control experiment was carried out with untreated PVA similarly.

### Example 11: Optimization of enzyme concentration for its immobilization on photoreactive PVA (figure 11)

The stock solution was prepared by taking 1 mg HRP in 100 ml PBS. Seven petri plates, each containing 50 mg photoreactive PVA and varied HRP concentration ie 2.5, 5, 10, 20, 40, 80 and 160 µl corresponding to 0.25, 0.5, 1.0, 2.0, 4.0, 8.0 and 16.0 µg of HRP respectively were irradiated in UV stratalinker at 365 nm for 30 minutes. The support were washed with washing buffer followed by the addition of 300 µl of substrate-dye buffer. Enzyme was assayed similarly as described in example 1. A control experiment was carried out with untreated PVA similarly.

### Example 12: Optimization of enzyme concentration for immobilization onto the microwave activated LCAA- CPG (figure 12)

Five petri plates each containing 50 mg photoreactive LCAA- CPG and 10, 25, 50, 100 or 200 µl HRP solution corresponding to 0.2, 0.5, 1.0, 2.0 or 4.0 µg respectively of HRP were irradiated in UV stratalinker at 365 nm for 20 minutes. Enzyme immobilized on photoreactive LCAA- CPG was then assayed as described in example 8. A control experiment was carried out with untreated LCAA- CPG.

### Example 13: Optimization of enzyme concentration for immobilization onto the microwave activated photoreactive alkylamino silica gel (figure 13)

Five petri plates each containing 50 mg microwave activated photoreactive alkylamino silica gel and 0.5, 1.0, 2.0, 4.0 or 6.0 µg of HRP were irradiated in an UV stratalinker at 365 nm for 20 minutes. Immobilized enzyme was assayed as described in example 8. A control experiment was carried out with untreated alkylamino silica gel in the similar way.

### Example 14: Optimization of microwave exposure time for attachment of photolinker to LCAA-CPG (table 1)

Four conical flasks, each containing 50 mg LCAA- CPG, 50 mg FNAB and 10 ml DMF were exposed to microwave for 30, 50, 60 and 70 seconds respectively. After the stipulated time, the support from each conical flask were washed separately by methanol dried and kept on four petridishes. HRP was then immobilized similarly as described in example 1. Control experiment was carried out with untreated LCAA- CPG beads similarly.

### Example 15: Optimization of activation time for LCAA- CPG at 37°C (table1)

Four conical flasks, each containing 50 mg LCAA- CPG, 50 mg FNAB and 10 ml DMF were stirred for 5, 10, 15 and 20 hours respectively at 37°C. After the stipulated time, the support from each conical flask were washed separately by methanol, dried and kept on a petridish. HRP was then immobilized and assayed similarly as described in example 1. A control experiment was carried out with untreated LCAA- CPG beads in the similar way.

### Example 16: Comparison between photochemical and thermochemical activation of PVA (table 2)

Photochemical activation of PVA - 250 mg PVA and 250 mg FNAB in 2500 µl methanol were mixed in a petriplate. After evaporating methanol, reaction mixture was irradiated for 60 minutes in UV stratalinker at 365 nm. After which support was washed with methanol and dried. Four petri plates, each containing 50 mg thermoreactive PVA were mixed with 2, 4, 8 and 16 µg HRP respectively and incubated at 37°C for 60 minutes. Enzyme was assayed as in example 1. A control experiment was carried out with untreated PVA in the similar way.

Thermochemical activation of PVA- Thermal activation of PVA and HRP immobilization onto this photoreactive PVA were carried out by using same amount of PVA, FNAB and HRP as above and according to procedure as described in example 3.

### Example 17: Determination of activity of immobilized HRP onto the photoreactive LCAA- CPG and alkylamino silica gel.

HRP was immobilized onto the photoreactive LCAA- CPG by irradiating reaction mixture (1 µg HRP/ 50 µl PBS and 50 mg photoreactive LCAA -CPG) in UV stratalinker for 20 minutes. Activity of immobilized HRP was determined by measuring the rate of color development at 490 nm, using dianisidine as hydrogen donor and hydrogen peroxide as substrate. Standard curve was drawn using different amount of HRP (0.5, 1, 2, 4, 8, 16, 32 and 64 ng). The absorbance was recorded at 1-minute intervals for 10 minutes and rate of change of absorbance per minute was determined. Activity of immobilized HRP onto the photoreactive alkylamino silica gel was determined as above.

### Example 18: Comparison between microwave and thermal activation of PVA.

PVA (50 mg) was mixed with FNAB (50 mg), toluene (5 ml) and 6.5 µl of 30% KOH and kept for stirring at room temperature for 1 hour. Similar reaction mixture was exposed to microwave radiation for 10 minutes. After the reaction the support was washed with methanol and dried. 50 mg photoreactive alkylamino silica gel prepared at room temperature was mixed with 1 µl of HRP in a petriplate and irradiated in UV stratalinker at 365 nm for 30 minutes. Similar experiment was conducted by the photoreactive PVA prepared by microwaves. Immobilized HRP assayed as described above. A control experiment was carried out with untreated PVA in the similar way.

**Table 1**

| **Time (seconds/hours)** | **Mode of Activation** | **+ FNAB** | **-FNAB** |
|---|---|---|---|
| 30 seconds | microwave | 0.823 | 0.215 |
| 50 seconds | microwave | 1.021 | 0.311 |
| 60 seconds | microwave | 1.722 | 0.320 |
| 70 seconds | microwave | 1.960 | 0.415 |
| 5 hours | 37°C | 0.885 | 0.337 |
| 10 hours | 37°C | 0.966 | 0.321 |
| 15 hours | 37°C | 0.998 | 0.331 |
| 20 hours | 37°C | 1.368 | 0.320 |

**Table 2**

| **HRP (µg)** | **Mode of activation of PVA** | | | |
|---|---|---|---|---|
| | **Thermoactivation** | | **Photoactivation** | |
| | **+L** | **-L** | **+L** | **-L** |
| 2 | 0.992 | 0.177 | 0.359 | 0.181 |
| 4 | 1.447 | 0.221 | 0.482 | 0.211 |
| 8 | 1.742 | 0.335 | 0.831 | 0.320 |
| 16 | 1.791 | 0.411 | 1.317 | 0.359 |

### Table 1. Efficacy studies of photoreactive LCAA- CPG prepared by microwave irradiation and by 37°C incubation in different time (Example 14, 15)

Photoreactive LCAA- CPG was prepared in different microwave exposure time (30, 50, 60 and 70 seconds) and different incubation time (5, 10, 15 and 20 hours) at 37°C. Photoreactive surface was checked by photoirradiating 1 µg of HRP with 50 mg support and assaying the immobilized enzyme colorimetrically.

### Table 2. Comparison of the efficacy of immobilization onto thermochemically and photochemically activated PVA (Example 16)

Photoreactive and thermoreactive PVAs were prepared by thermochemical and photochemical reactions respectively. Their efficacy of immobilization was compared by immobilizing different amounts of HRP (2, 4, 8 and 16 µg) onto them and subsequently assaying the immobilized enzyme.

### ADVANTAGES

1. A simple, rapid and efficient method is invented for the preparation of a photoreactive polymer and rapid immobilization of biomolecule by UV light on to this surface.
2. Any polymer having reactive nucleophilic group can be activated by the invented method.
3. Activation of polymer can be carried out in 50 seconds for amino bearing polymers and in 10 minutes for hydroxyl group alcohol bearing polymers by the invented method.
4. In the invented method, activation is carried out by microwave radiation.
5. The invented photoreactive surface can potentially be used for immobilization of biomolecules used in diagnostics, affinity chromatography, proteomics, gemomics, drug screening and related fields.

### REFRENCES

1. Krysteva, et. al., "Covalent binding of enzymes to synthetic membranes containing acrylamide units, using formaldehyde", Biotechnol. Appl. Biochem. 13, 106-111(1991).
2. Pandey, et.al., "Amperometric enzyme sensor for glucose based on graphite paste-modified electrodes", Appl. Biochem. Biotechnol. 33, 139-144 (1992).
3. Tiller, J. et. al., "A novel efficient enzyme-immobilization reaction on NH2 polymers by means of L- ascorbic acid", Appl. Biochem. 30, 155-162 (1992).
4. Guo, et. al., "Immobilization of glucose oxidase and peroxidase and their application in their application in flow injection analysis for glucose in serum", Appl. Biochem. Biotechnol. 23(1), 15-24 (1990).
5. Nakamura, et. al., "Immobilization of uricase on protamine bound to glass beads and its application to determination of uric acid", Anal. Biochem. 152(2), 386-390 (1986).
6. Tawa, et. al., " Immobilization of xanthine oxidase to controlled pore glass. Application to high performance liquid chromatography", Nucleic Acids Symp. Ser. 12, 107-110 (1983).
7. Stabel, et. al., "Anti-IgG immobilized controlled-pore glass. Thionyl chloride-activated succinamidopropyl-glass as a covalent immobilization matrix", Appl. Biotechnol. 36(2), 87-96 (1992).
8. de Queiroz, et. al., "Surface studies of albumin immobilized onto PE and PVC films", J. Biomater. Sci. Polym. 8, 667-681(1997).
9. Siephia, et. al., "Immobilization of enzymes on polypropylene bead surfaces by anhydrous ammonia gaseous plasma technique", J Biomed. Mater. Res. 22(5), 417-22 (1988).
10. Nahar, et al., "Light-induced activivation of an inert surface for covalent immobilization of a protein ligand", Anal. Biochem. 294, 148-153 (2001).
11. Naqvi, et. al, "Introduction of functional groups onto polypropylene and polyethylene surfaces for immobilization of enzymes", Anal. Biochem. 30, 74-78 (2002).
12. Bora,et. al, "Covalent immobilization of proteins onto photoactivated polystyrene microtiter plates for enzyme-linked immunosorbent assay procedures", J. Immunol. Methods. 268, 171 (2002)
13. Nahar, P. A method for photochemical activation of polymer surface and immobilization of biomolecules onto the activated surface. US patent pending.
14. Sigrist, et. al., "Light dependent, Covalent immobilization of biomolecules on 'inert'surface", Biotechnology 10, 1026-1028 (1992).

### OTHER REFERENCES

| U. S. Patent Documents | | |
|---|---|---|
| 5,427,779 | 1995 | H. Elsner et. al., |
| 6,033,784. | 2000 | M. Jacobsen, et. al. |
| 3,959,078 | 1976 | P.E. Guire |
| 5,002,582 | 1991 | P.E. Guire, et. al |

International Patent Application WO-A-02/14868 (Scientific and Industrial Research Council).

U.S. Patent Application US-A-2003/0228410 (P. Nahar).

## Claims

1. A method for preparation of photoreactive polymers with molecules immobilized thereon, said method comprising:
(a) reacting a polymer with a nucleophilic group with a photolinker molecule dissolved in a suitable solvent,
(b) washing the polymer by a suitable solvent followed by drying the washed polymer at ambient temperature to obtain a photoreactive polymer,
(c) adding a molecule dissolved in a suitable buffer onto the photoreactive polymer,
(d) subjecting the mixture to a source of photoirradiation for a period ranging from 2 minutes to 2 hours to immobilize the molecule on said photoreactive polymer, and
(e) washing the said polymer having immobilized molecule with a suitable buffer followed by drying the said polymer at the ambient temperature to obtain photoreactive polymers with molecules immobilized thereon.

2. A method as claimed in claim 1 wherein the photolinker has a thermochemical group selected from a group capable of forming a covalent bond with the nucleophilic group of the polymer.

3. A method as claimed in claim 2 wherein the thermochemical group of the photolinker is selected from the group consisting of aldehyde, carbonyl, isothiocyanate, halide, and isocyanate.

4. A method as claimed in claim 1 wherein the photolinker has a photochemical group which is photoreactive and is capable of forming a covalent bond in a photochemical reaction with the molecule without addition of any other reagent.

5. A method as claimed in claim 4 wherein the photochemical group of the photolinker is selected from the group consisting of precursors of carbene, precursors of nitrene and oxygen radical.

6. A method as claimed in claim 1 wherein the photolinker is 1- fluoro-2- nitro-4-azidobenzene (FNAB).

7. A method as claimed in claim 1 wherein the polymer comprises any polymer having amino, hydroxyl, thiol or any other reactive nucleophilic group.

8. A method as claimed in claim 1 wherein the polymer is selected from the group consisting of silica gel, long chain alkyl amino controlled pore glass, polyvinyl alcohol, aminopolystyrene and alkylamino silica gel.

9. A method as claimed in claim 1 wherein the solvent used in step (a) is selected from the group consisting of dimethyl formamide and toluene.

10. A method as claimed in claim 1 wherein step (a) is carried out in the presence of a catalyst.

11. A method as claimed in claim 10 wherein the catalyst comprises 30% KOH.

12. A method as claimed in claim 1 wherein the reaction in step (a) is carried out by exposing the mixture of photolinker, polymer and solvent to microwave radiation or by thermal incubation at 37°C.

13. A method as claimed in claim 12 wherein the microwave radiation exposure is carried out at a frequency of from 2000 Hz to 2600 Hz with a power output ranging from 100 watts to 1000 watts for a period ranging from 10 seconds to 20 minutes.

14. A method as claimed in claim 1 wherein the solvent used for washing the polymer obtained in step (a) comprises a solvent capable of dissolving the photolinker and its degraded products without distorting the polymer.

15. A method as claimed in claim 14 wherein the solvent used is selected from methanol and ethanol.

16. A method as claimed in claim 1 wherein the polymer is selected in the form of a bead, well, sheet, powder, stick, plate, strip or a tube.

17. A method as claimed in claim 1 wherein the molecule immobilized on the photoreactive polymer is selected from the group consisting of organic molecule, organic polymer, biomolecule and any molecule with C-H linkage.

18. A method as claimed in claim 17 wherein the biomolecule is selected from the group consisting of protein, nucleic acids, carbohydrate, oligonucleotides, enzyme, antigen, antibody and peptide.

19. A method as claimed in claim 18 wherein the enzyme is horse radish peroxidase.

20. A method as claimed in claim 1 wherein the source of photoenergy is selected from the group consisting of UV light, sunlight, flush light and laser beam.

21. A method as claimed in claim 1 wherein the UV light used as photoenergy source has a wavelength in the range of 300 nm to 400 nm and exposure to immobilize the molecule is carried out for a period in the range of 2 minutes to 2 hours.

22. A method as claimed in claim 1 wherein the reaction between nucleophilic group of the polymer surface and the photolinker is carried out in a microwave oven in presence of a solvent.

## Patentansprüche

1. Verfahren zum Herstellen von photoreaktiven Polymeren mit darauf immobilisierten Molekülen, Verfahren welches umfasst:
(a) ein Reagieren eines Polymers mit einer nukleophilen Gruppe mit einem Photoverbindungsmolekül, das in einem geeigneten Lösungsmittel aufgelöst ist,
(b) ein Waschen des Polymers mit einem geeigneten Lösungsmittel, gefolgt von einem Trocknen des gewaschenen Polymers bei Umgebungstemperatur, um ein photoreaktives Polymer zu erzielen,
(c) ein Hinzufügen eines in einem geeigneten Puffer aufgelösten Moleküls auf das photoreaktive Polymer,
(d) ein Unterziehen der Mischung einer Quelle einer Photobestrahlung während einer von 2 Minuten bis zu 2 Stunden reichenden Zeitdauer, um das Molekül auf dem photoreaktiven Polymer zu immobilisieren, und
(e) ein Waschen des Polymers mit dem immobilisierten Molekül mit einem geeigneten Puffer, gefolgt von einem Trocknen des Polymers bei Umgebungstemperatur, um photoreaktive Polymere mit darauf immobilisierten Molekülen zu erzielen.

2. Verfahren gemäß Anspruch 1, bei welchem das Photoverbindungsmittel eine thermochemische Gruppe aufweist, die ausgewählt ist aus einer Gruppe, die in der Lage ist, eine kovalente Bindung mit der nukleophilen Gruppe des Polymers zu bilden.

3. Verfahren gemäß Anspruch 2, bei welchem die thermochemische Gruppe des Photoverbindungsmittels ausgewählt ist aus der Gruppe bestehend aus Aldehyd, Carbonyl, Isothiocyanat, Halid und Isocyanat.

4. Verfahren gemäß Anspruch 1, bei welchem das Photoverbindungsmittel eine photochemische Gruppe aufweist, die photoreaktiv ist und die in der Lage ist, eine kovalente Bindung bei einer photochemischen Reaktion mit dem Molekül ohne Zugabe irgendeines anderen Reagensmittels zu bilden.

5. Verfahren gemäß Anspruch 4, bei welchem die photochemische Gruppe des Photoverbindungsmittels ausgewählt ist aus der Gruppe bestehend aus den Vorläufern von Carben, den Vorläufern von Nitren und einem Sauerstoffradikal.

6. Verfahren gemäß Anspruch 1, bei welchem das Photoverbindungsmittel 1-Fluor-2-nitro-4-azidobenzol (FNAB) ist.

7. Verfahren gemäß Anspruch 1, bei welchem das Polymer irgendein Polymer mit Amino, Hydroxyl, Thiol oder mit irgendeiner anderen reaktiven nukleophilen Gruppe umfasst.

8. Verfahren gemäß Anspruch 1, bei welchem das Polymer ausgewählt ist aus der Gruppe bestehend aus Kieselgel, langkettigem Alkylaminoglas mit kontrollierten Poren, Polyvinylalkohol, Aminopolystyrol und Alkylaminokieselgel.

9. Verfahren gemäß Anspruch 1, bei welchem das in dem Schritt (a) verwendete Lösungsmittel ausgewählt ist aus der Gruppe bestehend aus Dimethylformamid und Toluol.

10. Verfahren gemäß Anspruch 1, bei welchem der Schritt (a) in Gegenwart eines Katalysators durchgeführt wird.

11. Verfahren gemäß Anspruch 10, bei welchem der Katalysator 30 % KOH umfasst.

12. Verfahren gemäß Anspruch 1, bei welchem die Reaktion nach dem Schritt (a) durchgeführt wird, indem die Mischung aus dem Photoverbindungsmittel, dem Polymer und dem Lösungsmittel einer Mikrowellenstrahlung ausgesetzt wird oder durch eine thermische Inkubation bei 37°C erfolgt.

13. Verfahren gemäß Anspruch 12, bei welchem die Aussetzung gegenüber einer Mikrowellenstrahlung bei einer Frequenz von 2000 Hz bis 2600 Hz mit einer Leistungsabgabe in dem Bereich von 100 Watt bis 1000 Watt während einer Zeitdauer in dem Bereich von 10 Sekunden bis 20 Minuten durchgeführt wird.

14. Verfahren gemäß Anspruch 1, bei welchem das für das Waschen des bei dem Schritt (a) erzielten Polymers verwendete Lösungsmittel ein Lösungsmittel umfasst, welches in der Lage ist, das Photoverbindungsmittel und seine degradierten Produkte ohne Verzerrung des Polymers aufzulösen.

15. Verfahren gemäß Anspruch 14, bei welchem das verwendete Lösungsmittel ausgewählt ist unter Methanol und Ethanol.

16. Verfahren gemäß Anspruch 1, bei welchem das Polymer ausgewählt ist in der Form einer Perle, eines Schachtes, einer Folie, eines Pulvers, eines Stabes, einer Platte, eines Streifens oder eines Rohres.

17. Verfahren gemäß Anspruch 1, bei welchem das auf dem photoreaktiven Polymer immobilisierte Molekül ausgewählt ist aus der Gruppe bestehend aus einem organischen Molekül, einem organischen Polymer, einem Biomolekül und irgendeinem Molekül mit einer C-H Verbindung.

18. Verfahren gemäß Anspruch 17, bei welchem das Biomolekül ausgewählt ist aus der Gruppe bestehend aus Protein, Nukleinsäuren, Kohlenhydrat, Oligonukleotiden, Enzym, Antigen, Antikörper und Peptid.

19. Verfahren gemäß Anspruch 18, bei welchem das Enzym eine Rettichperoxidase ist.

20. Verfahren gemäß Anspruch 1, bei welchem die Quelle der Photoenergie ausgewählt ist aus der Gruppe bestehend aus UV Licht, Sonnenlicht, ebenem Licht und aus einem Laserstrahl.

21. Verfahren gemäß Anspruch 1, bei welchem das als Quelle für die Photoenergie verwendete UV-Licht eine Wellenlänge in dem Bereich von 300 nm bis 400 nm aufweist und das Aussetzen zum Immobilisieren des Moleküls während einer Zeitdauer in dem Bereich von 2 Minuten bis 2 Stunden durchgeführt wird.

22. Verfahren gemäß Anspruch 1, bei welchem die Reaktion zwischen der nukleophilen Gruppe der Polymeroberfläche und dem Photoverbindungsmittel in einem Mikrowellenofen in Gegenwart eines Lösungsmittels durchgeführt wird.

## Revendications

1. Procédé pour la préparation de polymères photoréactifs avec des molécules immobilisées sur ceux-ci, le procédé comprenant:
a) la réaction d'un polymère avec un groupe nucléophile avec une molécule de photolieur dissoute dans un solvant approprié,
b) le lavage du polymère par un solvant approprié suivi par le séchage du polymère lavé à la température ambiante pour obtenir un polymère photoréactif,
c) l'ajout d'une molécule dissoute dans un tampon approprié sur le polymère photoréactif,
d) l'exposition du mélange à une source de photoirradiation pendant une période variant de 2 minutes à 2 heures pour immobiliser la molécule sur ledit polymère photoréactif, et
e) le lavage dudit polymère possédant une molécule immobilisée avec un tampon approprié suivi par le séchage dudit polymère à la température ambiante pour obtenir des polymères photoréactifs avec des molécules immobilisées sur ceux-ci.

2. Procédé suivant la revendication 1, dans lequel le photolieur possède un groupe thermochimique choisi parmi un groupe capable de former une liaison covalente avec le groupe nucléophile du polymère.

3. Procédé suivant la revendication 2, dans lequel le groupe thermochimique du photolieur est choisi parmi le groupe constitué d'aldéhyde, de carbonyle, d'isothiocyanate, d'halogénure et d'isocyanate.

4. Procédé suivant la revendication 1, dans lequel le photolieur possède un groupe photochimique qui est photoréactif et qui est capable de former une liaison covalente dans une réaction photochimique avec la molécule sans l'ajout d'aucun autre réactif.

5. Procédé suivant la revendication 4, dans lequel le groupe photochimique du photolieur est choisi parmi le groupe constitué de précurseurs de carbène, de précurseurs de nitrène et d'oxygène radicalaire.

6. Procédé suivant la revendication 1, dans lequel le photolieur est le 1-fluoro-2-nitro-4-azidobenzène (FNAB).

7. Procédé suivant la revendication 1, dans lequel le polymère comprend tout polymère possédant un groupe amino, hydroxyle, thiol ou un quelconque autre groupe nucléophile réactif.

8. Procédé suivant la revendication 1, dans lequel le polymère est choisi parmi le groupe constitué de gel de silice, de verre à pores régulés d'alkylamino à longue chaîne, de polyvinylalcool, d'aminopolystyrène et de gel de silice d'alkylamino.

9. Procédé suivant la revendication 1, dans lequel le solvant utilisé dans l'étape (a) est choisi parmi le groupe constitué de diméthylformamide et de toluène.

10. Procédé suivant la revendication 1, dans lequel l'étape (a) est réalisée en présence d'un catalyseur.

11. Procédé suivant la revendication 10, dans lequel le catalyseur comprend 30% de KOH.

12. Procédé suivant la revendication 1, dans lequel la réaction dans l'étape (a) est réalisée par l'exposition du mélange de photolieur, de polymère et de solvant à un rayonnement micro-onde ou par une incubation thermique à 37°C.

13. Procédé suivant la revendication 12, dans lequel l'exposition à un rayonnement micro-onde est réalisée à une fréquence de 2000 Hz à 2600 Hz avec une puissance de sortie variant de 100 Watts à 1000 Watts pendant une période variant de 10 secondes à 20 minutes.

14. Procédé suivant la revendication 1, dans lequel le solvant utilisé pour le lavage du polymère obtenu dans l'étape (a) comprend un solvant capable de dissoudre le photolieur et ses produits dégradés sans distorsion du polymère.

15. Procédé suivant la revendication 14, dans lequel le solvant utilisé est choisi parmi le méthanol et l'éthanol.

16. Procédé suivant la revendication 1, dans lequel le polymère est choisi sous la forme d'une bille, d'un puits, d'une feuille, d'une poudre, d'un bâton, d'une plaque, d'une bande ou d'un tube.

17. Procédé suivant la revendication 1, dans lequel la molécule immobilisée sur le polymère photoréactif est choisie parmi le groupe constitué de molécule organique, de polymère organique, de biomolécule et de toute molécule avec une liaison C-H.

18. Procédé suivant la revendication 17, dans lequel la biomolécule est choisie parmi le groupe constitué de protéine, d'acides nucléiques, de carbohydrate, d'oligonucléotides, d'enzyme, d'antigène, d'anticorps et de peptide.

19. Procédé suivant la revendication 18, dans lequel l'enzyme est une peroxydase de raifort.

20. Procédé suivant la revendication 1, dans lequel la source de photoénergie est choisie parmi le groupe constitué de lumière UV, de lumière du soleil, de feu encastré et de faisceau laser.

21. Procédé suivant la revendication 1, dans lequel la lumière UV utilisée comme source de photoénergie possède une longueur d'onde dans l'intervalle de 300 nm à 400 nm et l'exposition pour immobiliser la molécule est réalisée pendant une période dans l'intervalle de 2 minutes à 2 heures.

22. Procédé suivant la revendication 1, dans lequel la réaction entre le groupe nucléophile de la surface du polymère et le photolieur est réalisée dans un four à micro-onde en présence d'un solvant.
